# EUROPEAN PATENT APPLICATION

(11) **EP 2 080 492 A1**
(43) Date of publication of application: **22.07.2009**
(21) Application number: 09250106.3
(22) Date of filing: 16.01.2009
(51) Int. Cl.: A61F 5/00

(54) **Optimizing the operation of a restriction system**

(30) Priority: 17.01.2008 US 15634
(71) Applicant: Ethicon Endo-Surgery, Inc., Cincinnati, OH 45242 (US)
(72) Inventor: Ortiz, Mark S., Milford Ohio 45150 (US); Dlugos, Daniel F., Jr., Middletown Ohio 45044 (US); Marcotte, Amy L., Mason Ohio 45040 (US)
(74) Representative: Tunstall, Christopher Stephen

(57) **Abstract**

Methods and devices for optimizing the operation of a restriction system for forming a restriction in a patient are disclosed. In one exemplary embodiment, a method for optimizing the operation of a gastric restriction system includes providing an implantable restriction system for forming a restriction in a patient, determining an optimum value of a control parameter of the restriction system, and maintaining the control parameter at the optimum value such that a result parameter of the restriction system is substantially convergent as a function of time.
Determining an optimum value of a control parameter can generally include adjusting the restriction device, determining the value of the control parameter to be optimized, and repeating the steps of adjusting the restriction device and determining the value of the control parameter until the control parameter is substantially convergent as a function of time.

## Description

### FIELD OF THE INVENTION

The present invention relates to methods and devices for optimizing the operation of a restriction system.

### BACKGROUND OF THE INVENTION

Obesity is becoming a growing concern, particularly in the United States, as the number of obese people continues to increase, and more is learned about the negative health effects of obesity. Morbid obesity, in which a person is 100 pounds or more over ideal body weight, in particular poses significant risks for severe health problems. Accordingly, a great deal of attention is being focused on treating obese patients. One method of treating morbid obesity has been to place a restriction device, such as an elongated band, about the upper portion of the stomach. Gastric bands have typically comprised a fluid-filled elastomeric balloon with fixed endpoints that encircles the stomach just inferior to the esophageal-gastric junction to form a small gastric pouch above the band and a reduced stoma opening in the stomach. When fluid is infused into the balloon, the band expands against the stomach creating a food intake restriction or stoma in the stomach. To decrease this restriction, fluid is removed from the band. The effect of the band is to reduce the available stomach volume and thus the amount of food that can be consumed before becoming "full."

Food restriction devices have also comprised mechanically adjusted bands that similarly encircle the upper portion of the stomach. These bands include any number of resilient materials or gearing devices, as well as drive members, for adjusting the bands. Additionally, gastric bands have been developed that include both hydraulic and mechanical drive elements. An example of such an adjustable gastric band is disclosed in U.S. Pat. No. 6,067,991, entitled "Mechanical Food Intake Restriction Device" which issued on May 30, 2000, and is incorporated herein by reference. It is also known to restrict the available food volume in the stomach cavity by implanting an inflatable elastomeric balloon within the stomach cavity itself. The balloon is filled with a fluid to expand against the stomach walls and, thereby, decrease the available food volume within the stomach.

With each of the above-described food restriction devices, safe, effective treatment requires that the device be regularly monitored and adjusted to vary the degree of restriction applied to the stomach. With banding devices, the gastric pouch above the band will substantially increase in size following the initial implantation. Accordingly, the stoma opening in the stomach must initially be made large enough to enable the patient to receive adequate nutrition while the stomach adapts to the banding device. As the gastric pouch increases in size, the band may be adjusted to vary the stoma size. In addition, it is desirable to vary the stoma size in order to accommodate changes in the patient's body or treatment regime, or in a more urgent case, to relieve an obstruction or severe esophageal dilatation. Traditionally, adjusting a hydraulic gastric band required a scheduled clinician visit during which a Huber needle and syringe were used to penetrate the patient's skin and add or remove fluid from the balloon via the injection port. More recently, implantable pumps have been developed which enable non-invasive adjustments of the band. An external programmer communicates with the implanted pump using telemetry to control the pump. During a scheduled visit, a physician places a handheld portion of the programmer near the gastric implant and transmits power and command signals to the implant. The implant in turn adjusts the fluid levels in the band and transmits a response command to the programmer.

During these gastric band adjustments, it has been difficult to determine how the adjustment is proceeding, and whether the adjustment will have the intended effect. In an attempt to determine the efficacy of an adjustment, some physicians have utilized fluoroscopy with a Barium swallow as the adjustment is being performed. However, fluoroscopy is both expensive and undesirable due to the radiation doses incurred by both the physician and patient. Other physicians have instructed the patient to drink a glass of water during or after the adjustment to determine whether the water can pass through the adjusted stoma. The water method, however, only assures that the patient is not obstructing, and does not provide any information about the efficacy of the adjustment. Oftentimes, a physician may simply adopt a "try as you go" method based upon their prior experience, and the results of an adjustment may not be discovered until hours or days later, when the patient experiences a complete obstruction to the stomach cavity, or the band induces erosion of the stomach tissue due to excessive interface pressures against the band.

It is often desirable to collect data concerning the operation of the restriction system as well as concerning the physiological characteristics of the patient. Thus, some restriction systems are equipped with a variety of sensors that can be configured to collect and transmit data that is useful for adjustment, diagnostic, monitoring, and other purposes. However, even these sensor equipped restriction systems require the physician to perform a series of adjustments to the system that often involve trial and error.

Accordingly, methods and devices are provided for use with a gastric restriction system, and in particular for optimizing the operation of a restriction system.

### SUMMARY OF THE INVENTION

The present invention generally provides devices and methods for optimizing the operation of a restriction system for forming a restriction in a patient. In one exemplary embodiment, a method for optimizing the operation of a gastric restriction system includes providing an implantable restriction system for forming a restriction in a patient, determining an optimum value of a control parameter of the restriction system, and maintaining the control parameter at the optimum value such that a result parameter of the restriction system has a substantial convergence as a function of time. The implantable restriction system of the method can have a variety of configurations. In general, the restriction system can include an adjustable restriction device that is configured to form a restriction in a patient.

Determining an optimum value of a control parameter can generally include adjusting the restriction device, determining the value of the control parameter to be optimized, and repeating the steps of adjusting the restriction device and determining the value of the control parameter until the control parameter is substantially convergent as a function of time (i.e., until the value of the control parameter substantially converges on a value over time). In one exemplary embodiment, determining the optimum value of a control parameter can further include detecting a value of the control parameter and comparing the detected value to a previously determined value of the control parameter.

If the detected value of the control parameter and the previously determined value of the control parameter are not substantially equal, the restriction device can be adjusted. A number of factors can affect the adjustment of the band. For example, the operating parameter chosen by the physician to be the control parameter, the measured value of the control parameter, and how the control parameter is measured can all influence the adjustment of the band. In one exemplary embodiment, if the detected measurement of the control parameter is less than the previously determined value of the control parameter, the restriction device can be tightened. The restriction device can generally be tightened by increasing the pressure within the restriction system. Alternatively, in another exemplary embodiment, if the detected measurement of the control parameter is greater than the previously determined value of the control parameter, the restriction device can be loosened. In one embodiment, the restriction device can be loosened by decreasing the pressure within the restriction system. As indicated above, several factors can affect the adjustment of the band. Thus, a detected value of the control parameter that is greater than a previously determined value of the control parameter does not always result in a loosening of the restriction device. Similarly, a detected value of a control parameter that is less than a previously determined value of the control parameter does not always result in a tightening of the restriction device.

In general, a control parameter can represent an operational parameter of the implantable restriction system that can be directly controlled by a physician via adjustment of the adjustable restriction device. Examples of control parameters include, but are not limited to, a pressure within the restriction system, a peristaltic pulse event or frequency, a peristaltic pulse width, a peristaltic pulse duration, a peristaltic pulse amplitude, and a flow rate of a bolus into the stomach. A result parameter generally represents an output result of the implantable restriction system that can be indirectly controlled by a physician via adjustment of the adjustable restriction device. Examples of result parameters include, but are not limited to, the body mass index of the patient, the weight of the patient, the change in weight of the patient, and percent excess weight lost by the patient.

A detected value of the control parameter that is substantially equal to a pre-determined value of the control parameter can include variations in the detected value of the control parameter in the range of about 5-10%. A control parameter that substantially converges on a value over time can include variations in the value of the control parameter in the range of about 5-10%. Similar to the control parameter, a result parameter that substantially converges as a function of time can include variations in the value of the result parameter in the range of about 5-10%.

The following is a non-exhaustive list of embodiments of the invention that may be claimed in this application or in subsequently filed divisional applications:
1. A method for determining an optimum control parameter of a restriction system for forming a restriction in a patient, comprising:
   providing an implantable restriction system for forming a restriction in a patient, the system including an adjustable restriction device configured to form a restriction in a patient;
   adjusting the restriction device;
   determining the value of a control parameter of the restriction system; and
   repeating the steps of adjusting the restriction device and determining the value of the control parameter until the control parameter is substantially convergent as a function of time.
2. The method of embodiment 1, further comprising comparing the determined value of the control parameter to a previously determined value of the control parameter.
3. The method of embodiment 2, wherein the restriction device is adjusted if the determined value of the control parameter is not substantially equal to the previously determined value of the control parameter.
4. The method of embodiment 3, wherein substantially equal includes variations in the value of the control parameter in the range of about 5-10%.
5. The method of embodiment 2, wherein adjusting the restriction device includes tightening the restriction device if the determined value of the control parameter is less than the previously determined value of the control parameter.
6. The method of embodiment 2, wherein adjusting the restriction device includes loosening the restriction device if the determined value of the control parameter is greater than the previously determined value of the control parameter.
7. The method of embodiment 1, wherein substantially convergent includes variations in the value of the control parameter in the range of about 5-10% over time.
8. The method of embodiment 1, wherein the control parameter is the pressure within the restriction system.
9. The method of embodiment 1, wherein the control parameter is at least one of the peristaltic pulse event, the peristaltic pulse width, the peristaltic pulse duration, the peristaltic pulse amplitude, and the flow rate of a bolus into the stomach.
10. A method for optimizing the operation of a restriction system for forming a restriction in a patient, comprising:
   providing an implantable restriction system for forming a restriction in a patient, the system including an adjustable restriction device configured to form a restriction in a patient;
   determining an optimum value of a control parameter of the restriction system by adjusting the restriction device until the control parameter is substantially convergent as a function of time; and
   maintaining the control parameter at the optimum value such that a result parameter of the restriction system is substantially convergent as a function of time.
11. The method of embodiment 10, wherein determining the optimum value of the control parameter includes detecting a value of the control parameter and comparing the detected value to a previously determined value of the control parameter.
12. The method of embodiment 11, wherein adjusting the restriction device includes tightening the restriction device if the detected value of the control parameter is less than the previously determined value of the control parameter.
13. The method of claim 11, wherein adjusting the restriction device includes loosening the restriction device if the detected value of the control parameter is greater than the previously determined value of the control parameter.
14. The method of embodiment 10, wherein substantially convergent includes variations in the value of the control parameter in the range of about 5-10% over time.
15. The method of embodiment 10, wherein substantially convergent includes variations in the value of the result parameter in the range of about 5-10% over time.
16. The method of embodiment 10, wherein the control parameter is the pressure within the restriction system.
17. The method of embodiment 10, wherein the control parameter is at least one of the peristaltic pulse event, the peristaltic pulse width, the peristaltic pulse duration, the peristaltic pulse amplitude, and the flow rate of a bolus into the stomach.
18. The method of embodiment 10, wherein the result parameter is at least one of the body mass index of the patient, the weight of the patient, the weight change of the patient, and the percent excess weight lost by the patient.
19. A method for optimizing the operation of a restriction system for forming a restriction in a patient, comprising:
   providing an implantable restriction system for forming a restriction in a patient, the system including an adjustable restriction device configured to form a restriction in a patient;
   determining an optimum value of a control parameter of the restriction system by adjusting the restriction device until the control parameter substantially converges on a value over time; and
   maintaining the control parameter at the optimum value such that a result parameter of the restriction system has a substantial convergence over time.
20. The method of embodiment 19, wherein determining the optimum value of the control parameter includes detecting a value of the control parameter and comparing the detected value to a previously determined value of the control parameter.
21. The method of embodiment 20, wherein adjusting the restriction device includes tightening the restriction device if the detected value of the control parameter is less than the previously determined value of the control parameter.
22. The method of embodiment 20, wherein adjusting the restriction device includes loosening the restriction device if the detected value of the control parameter is greater than the previously determined value of the control parameter.
23. The method of embodiment 19, wherein substantially converges includes variations in the value of the control parameter in the range of about 5-10% over time.
24. The method of embodiment 19, wherein substantial convergence includes variations in the value of the result parameter in the range of about 5-10% over time.
25. The method of embodiment 19, wherein the control parameter is at least one of the pressure within the restriction system, the peristaltic pulse event, the peristaltic pulse width, the peristaltic pulse duration, the peristaltic pulse amplitude, and the flow rate of a bolus into the stomach.
26. The method of embodiment 19, wherein the result parameter is at least one of the body mass index of the patient, the weight of the patient, the weight change of the patient, and the percent excess weight lost by the patient.

### BRIEF DESCRIPTION OF THE DRAWINGS

The invention will be more fully understood from the following detailed description taken in conjunction with the accompanying drawings, in which:

FIG. 1A is a perspective view of one embodiment of a food intake restriction system;

FIG. 1B is perspective view of one embodiment of a restriction system;

FIG. 2A is a perspective view of the gastric band of the restriction system shown in FIG. 1B;

FIG. 2B is a perspective view of the gastric band shown in FIG. 2A as applied to the gastro-esophageal junction of a patient;

FIG. 3 is a perspective view of the fluid injection port of the restriction system shown in FIG. 1B;

FIG. 4 is a perspective view of another embodiment of a restriction system;

FIG. 5 is a perspective view of the sensor housing shown in FIG. 1A;

FIG. 6 is a schematic of an embodiment of a variable resistance circuit for the pressure sensor of FIG. 5;

FIG. 7 is a block diagram of one embodiment of a pressure management system for use in conjunction with the restriction system shown in FIG. 4;

FIG. 8 is a flow diagram of one embodiment of a method for optimizing the operation of a restriction system for forming a restriction in a patient;

FIG. 9 is a flow diagram of one embodiment of a method for determining an optimum control parameter of a restriction system for forming a restriction in a patient;

FIG. 10 is a flow diagram of one embodiment of a method for optimizing the operation of a restriction system for forming a restriction in a patient;

FIG. 11 is a flow diagram of one embodiment of a method for determining an optimum control parameter of a restriction system for forming a restriction in a patient;

FIG. 12 is a flow diagram of one embodiment of a method for returning a control parameter of a restriction system for forming a restriction in a patient to an optimum value;

FIG. 13A is a graphical representation of the value of a control parameter of a restriction system as a function of time;

FIG. 13B is a graphical representation of the value of a result parameter of the restriction system of FIG. 13A as a function of time;

FIG. 14A is a graphical representation of the value of a control parameter of a restriction system as a function of time; and

FIG. 14B is a graphical representation of the value of a result parameter of the restriction system of FIG. 14A as a function of time.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention generally provides methods and devices for optimizing the operation of a restriction system for forming a restriction in a patient. In one exemplary embodiment, the method includes providing an implantable restriction system for forming a restriction in a patient, determining an optimum value of a control parameter of the restriction system, and maintaining the control parameter at the optimum value such that a result parameter of the restriction system is substantially convergent as a function of time. In one embodiment, determining an optimum value of a control parameter of the restriction system can include adjusting the restriction device, determining the value of a control parameter of the restriction system, and repeating the steps of adjusting the restriction device and determining the value of the control parameter until the control parameter is substantially convergent as a function of time.

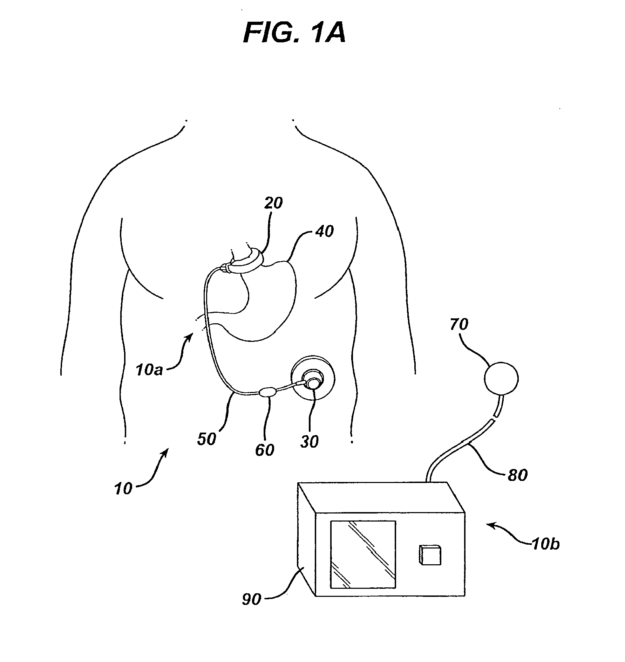

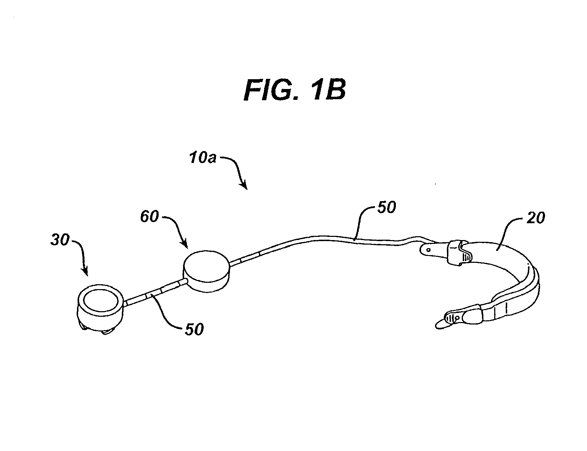

While the present invention can be used with a variety of restriction systems known in the art, FIG. 1A illustrates one exemplary embodiment of a food intake restriction system 10 in use in a patient. As shown, the system 10 generally includes an implantable portion 10a and an external portion 10b. FIG. 1B illustrates the implantable portion 10a outside of a patient. As shown, the implantable portion 10a includes an adjustable gastric band 20 that is configured to be positioned around the upper portion of a patient's stomach 40 and an injection port housing 30 that is fluidly coupled to the adjustable gastric band 20, e.g., via a catheter 50. The injection port 30 is adapted to allow fluid to be introduced into and removed from the gastric band 20 to thereby adjust the size of the band 20 and thus the pressure applied to the stomach 40. The injection port 30 can thus be implanted at a location within the body that is accessible through tissue. Typically, injection ports are positioned in the lateral subcostal region of the patient's abdomen under the skin and layers of fatty tissue. Surgeons also typically implant injection ports on the sternum of the patient.

The internal portion 10a can also include a sensing or measuring device that is in fluid communication with the closed fluid circuit in the implantable portion 10a. In one embodiment, the sensing device is a pressure sensing device configured to measure the fluid pressure of the closed fluid circuit. While the pressure measuring device can have various configurations and can be positioned anywhere along the internal portion 10a, including within the injection port 30 and as described further below, in the illustrated embodiment the pressure measuring device is in the form of a pressure sensor that is disposed within a sensor housing 60 positioned adjacent to the injection port 30. The catheter 50 can include a first portion that is coupled between the gastric band 20 and the pressure sensor housing 60 and a second portion that is coupled between the pressure sensor housing 60 and the injection port 30. While it is understood that the sensing device can be configured to obtain data relating to one or more relevant parameters, generally it will be described herein in a context of a pressure sensing device.

In addition to sensing pressure of fluid within the internal portion 10a as described herein, pressure of fluid within the esophagus and/or the stomach 40 can also be sensed using any suitable device, such as an endoscopic manometer. By way of non-limiting example, such fluid pressure measurements can be compared against measured pressure of fluid within the internal portion 10a before, during, and/or after adjustment of pressure within the internal portion 10a. Other suitable uses for measured pressure within the esophagus and/or the stomach 40 will be appreciated by those skilled in the art.

As further shown in FIG. 1A, the external portion 10b generally includes a data reading device 70 that is configured to be positioned on the skin surface above the pressure sensor housing 60 (which can be implanted beneath thick tissue, e.g., over 10 cm thick) to
non-invasively communicate with the pressure sensor housing 60 and thereby obtain pressure measurements. The data reading device 70 can optionally be electrically coupled (wirelessly or wired, as in this embodiment via an electrical cable assembly 80) to a control box 90 that can display the pressure measurements, other data obtained from the data reading device 70, and/or data alerts, as discussed further below. While shown in this example as located local to the patient, the control box 90 can be at a location local to or remote from the patient.

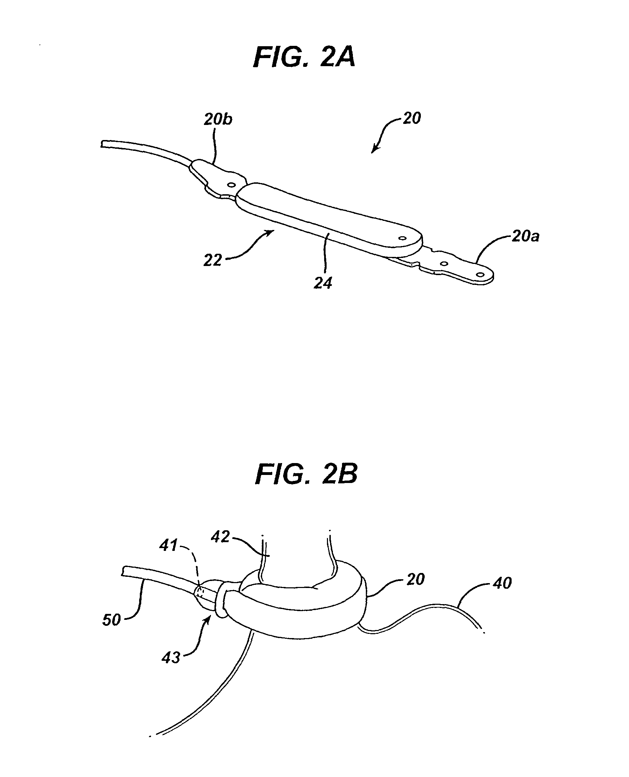

FIG. 2A shows the gastric band 20 in more detail. While the gastric band 20 can have a variety of configurations, and various gastric bands currently known in the art can be used with the present invention, in the illustrated embodiment the gastric band 20 has a generally elongate shape with a support structure 22 having first and second opposite ends 20a, 20b that can be formed in a loop such that the ends are secured to each other. Various mating techniques can be used to secure the ends 20a, 20b to one another. In the illustrated embodiment, the ends 20a, 20b are in the form of straps that mate together, with one laying on top of the other. In another embodiment, illustrated, for example, in FIGS. 1B and 2B, a support structure at one end of the gastric band 20 can include an opening through which the other end of the gastric band 20 can feed through to secure the ends to one another. The gastric band 20 can also include a variable volume member, such as an inflatable balloon 24, that is disposed or formed on one side of the support structure 22 and that is configured to be positioned adjacent to tissue. The balloon 24 can expand or contract against the outer wall of the stomach to form an adjustable stoma for controllably restricting food intake into the stomach.

A person skilled in the art will appreciate that the gastric band can have a variety of other configurations. Moreover, the various methods and devices disclosed herein have equal applicability to other types of implantable bands. For example, bands are used for the treatment of fecal incontinence, as described in U.S. Pat. No. 6,461,292 which is hereby incorporated by reference. Bands can also be used to treat urinary incontinence, as described in U.S. Publication No. 2003/0105385 which is hereby incorporated by reference. Bands can also be used to treat heartburn and/or acid reflux, as disclosed in U.S. Pat. No. 6,470,892 which is hereby incorporated by reference. Bands can also be used to treat impotence, as described in U.S. Publication No. 2003/0114729 which is hereby incorporated by reference.

FIG. 2B shows the adjustable gastric band 20 applied about the gastro-esophageal junction of a patient. As shown, the band 20 at least substantially encloses the upper portion of the stomach 40 near the junction with the patient's esophagus 42. After the band 20 is implanted, preferably in the deflated configuration wherein the band 20 contains little or no fluid, the band 20 can be inflated, e.g., using saline, to decrease the size of the stoma opening. A person skilled in the art will appreciate that various techniques, including mechanical and electrical techniques, can be used to adjust the band 20. FIG. 2B also shows an alternate location of a sensing device 41, disposed in a buckle 43 of the band 20.

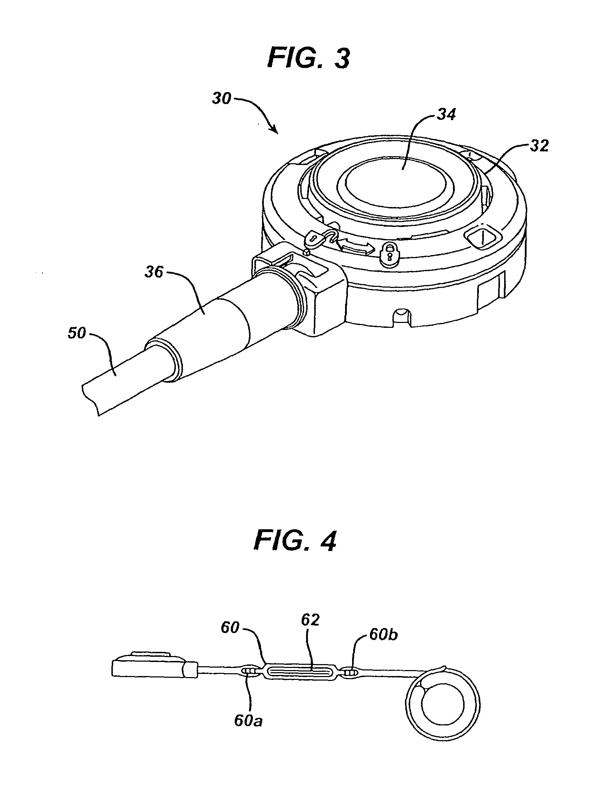

The fluid injection port 30 can also have a variety of configurations. In the embodiment shown in FIG. 3, the injection port 30 has a generally cylindrical housing with a distal or bottom surface and a perimeter wall extending proximally from the bottom surface and defining a proximal opening 32. The proximal opening 32 can include a needle-penetrable septum 34 extending there across and providing access to a fluid reservoir (not visible in FIG. 3) formed within the housing. The septum 34 is preferably placed in a proximal enough position such that the depth of the reservoir is sufficient enough to expose the open tip of a needle, such as a Huber needle, so that fluid transfer can take place. The septum 34 is preferably arranged so that it will self seal after being punctured by a needle and the needle is withdrawn. As further shown in FIG. 3, the port 30 can further include a catheter tube connection member 36 that is in fluid communication with the reservoir and that is configured to couple to a catheter (e.g., the catheter 50). A person skilled in the art will appreciate that the housing can be made from any number of materials, including stainless steel, titanium, or polymeric materials, and the septum 34 can likewise be made from any number of materials, including silicone.

The reading device 70 can also have a variety of configurations, and one exemplary pressure reading device is disclosed in more detail in commonly-owned U.S. Publication No. 2006/0189888 and U.S. Publication No. 2006/0199997, which are hereby incorporated by reference. In general, the reading device 70 can non-invasively measure the pressure of the fluid within the implanted portion 10a even when the pressure sensing device is implanted beneath thick (at least over 10 cm) subcutaneous fat tissue. The physician can hold the reading device 70 against the patient's skin near the location of the sensor housing 60 and/or other pressure sensing device location(s), obtain sensed pressure data and possibly other information as discussed herein, and observe the pressure reading (and/or other data) on a display on the control box 90. The data reading device 70 can also be removably attached to the patient, as discussed further below, such as during a prolonged examination, using straps, adhesives, and other well-known methods. The data reading device 70 can operate through conventional cloth or paper surgical drapes, and can also include a disposal cover (not shown) that may be replaced for each patient.

As indicated above, the system 10 can also include one or more sensors for monitoring the operation of the gastric restriction system 10. The sensor(s) can be configured to measure various operational parameters of the system 10 including, but not limited to, a pressure within the system, a temperature within the system, a peristaltic pulse event or frequency, the peristaltic pulse width, the peristaltic pulse duration, and the peristaltic pulse amplitude. In one exemplary embodiment, the system can include a sensor in the form of a pressure measuring device that is in communication with the closed fluid circuit and that is configured to measure the fluid pressure within the system, which corresponds to the amount of restriction applied by the adjustable gastric band to the patient's stomach. As is explained below in detail, measuring the fluid pressure, or any other control parameter of the system, can enable a physician to evaluate the performance of the restriction system. In the illustrated embodiment, shown in FIG. 4, the pressure measuring device is in the form of a pressure sensor 62 disposed within the sensor housing 60. The pressure measuring device can, however, be disposed anywhere within the closed hydraulic circuit of the implantable portion, and various exemplary locations and configurations are disclosed in more detail in commonly-owned U.S. Publication No. 2006/0211913 entitled "Non-Invasive Pressure Measurement In a Fluid Adjustable Restrictive Device," filed on March 7, 2006 and hereby incorporated by reference. In general, the illustrated sensor housing 60 includes an inlet 60a and an outlet 60b that are in fluid communication with the fluid in the implantable portion 10a. An already-implanted catheter 50 can be retrofitted with the sensor housing 60, such as by severing the catheter 50 and inserting barbed connectors (or any other connectors, such as clamps, clips, adhesives, welding, etc.) into the severed ends of the catheter 50. The sensor 62 can be disposed within the housing 60 and be configured to respond to fluid pressure changes within the hydraulic circuit and convert the pressure changes into a usable form of data.

Various pressure sensors known in the art can be used as the pressure sensor 62, such as a wireless pressure sensor provided by CardioMEMS, Inc. of Atlanta, Georgia, though a suitable MEMS pressure sensor may be obtained from any other source, including but not limited to Integrated Sensing Systems, Inc. (ISSYS) of Ypsilanti, Michigan and Remon Medical Technologies, Inc. of Waltham, Massachusetts. One exemplary MEMS pressure sensor is described in U.S. Patent No. 6,855,115, the disclosure of which is incorporated by reference herein for illustrative purposes only. It will also be appreciated by a person skilled in the art that suitable pressure sensors can include, but are not limited to, capacitive, piezoresistive, silicon strain gauge, or ultrasonic (acoustic) pressure sensors, as well as various other devices capable of measuring pressure.

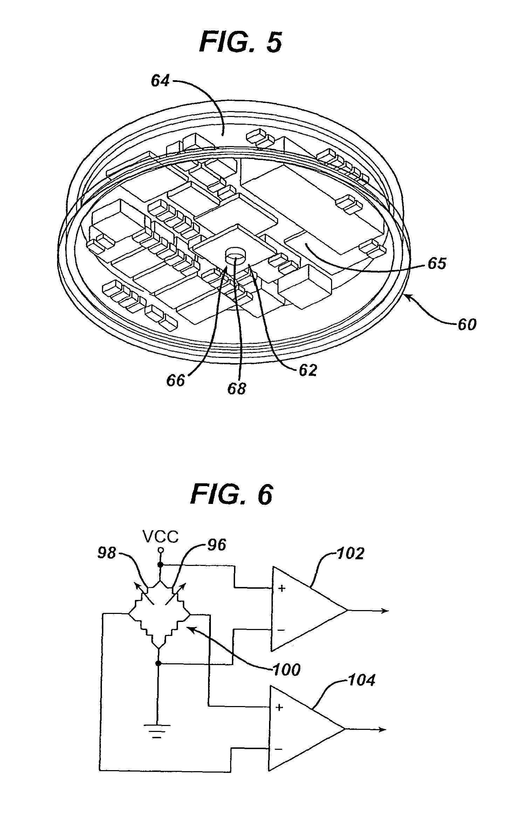

One embodiment of a configuration of the sensor housing 60 having the sensor 62 disposed within it is shown in FIG. 5. The sensor housing 60 in this example includes a motherboard that can serve as a hermetic container to prevent fluid from contacting any elements disposed within the sensor housing 60, except as discussed for the sensor 62. The sensor housing 60 can be made from any biocompatible material appropriate for use in a body, such as a polymer, biocompatible metal, and other similar types of material. Furthermore, the sensor housing 60 can be made from any one or more of transparent (as shown in FIG. 5), opaque, semi-opaque, and radio-opaque materials. A circuit board 64 including, among other elements, a microcontroller 65 (e.g., a processor), can also be disposed within the housing 60 to help process and communicate pressure measurements gathered by the sensor 62, and also possibly other data related to the band 20. As further discussed below, the circuit board 64 can also include a transcutaneous energy transfer (TET)/telemetry coil and a capacitor. Optionally, a temperature sensor can be integrated into the circuit board 64. The microcontroller 65, the TET/telemetry coil, the capacitor, and/or the temperature sensor can be in communication via the circuit board 64 or via any other suitable component(s). The TET/telemetry coil and capacitor can collectively form a tuned tank circuit for receiving power from the external portion 10b and transmitting pressure measurements to a pressure reading device, e.g., the reading device 70. Moreover, to the extent that a telemetry component associated with the pressure sensor 62 is unable to reach a telemetry device external to the patient without some assistance, such assistance can be provided by any suitable number of relays (not shown) or other devices.

Fluid can enter the sensor housing 60 through an opening 66 located anywhere on the housing's surface (here, its bottom surface) and come into contact with a pressure sensing surface 68 of the sensor 62. The sensor 62 is typically hermetically sealed to the motherboard such that fluid entering the opening 66 cannot infiltrate and affect operation of the sensor 62 except at the pressure sensing surface 68. The sensor 62 can measure the pressure of fluid coming into contact with the pressure sensing surface 68 as fluid flows in and out of the opening 66. For example, the pressure sensing surface 68 can include a diaphragm having a deformable surface such that when fluid flows through the opening 66, the fluid impacts the surface of the diaphragm, causing the surface to mechanically displace. The mechanical displacement of the diaphragm can be converted to an electrical signal by a variable resistance circuit including a pair of variable resistance, silicon strain gauges. One strain gauge can be attached to a center portion of diaphragm to measure the displacement of the diaphragm, while the second, matched strain gauge can be attached near the outer edge of diaphragm. The strain gauges can be attached to the diaphragm with adhesives or can be diffused into the diaphragm structure. As fluid pressure within band 20 fluctuates, the surface of the diaphragm can deform up or down, thereby producing a resistance change in the center strain gauge.

One embodiment of a variable resistance circuit for the sensor 62 is shown in FIG. 6.
The circuit includes first and second strain gauges 96, 98 that form the top two resistance elements of a half-compensated, Wheatstone bridge circuit 100. As the first strain gauge 96 reacts to the mechanical displacements of the sensor's diaphragm, the changing resistance of the first gauge 96 changes the potential across the top portion of the bridge circuit 100. The second strain gauge 98 is matched to the first strain gauge 96 and athermalizes the Wheatstone bridge circuit 100. First and second differential amplifiers 102, 104 are connected to the bridge circuit 100 to measure the change in potential within the bridge circuit 100 due to the variable resistance strain gauges 96, 98. In particular, the first differential amplifier 102 measures the voltage across the entire bridge circuit 100, while the second differential amplifier 104 measures the differential voltage across the strain gauge half of bridge circuit 100. The greater the differential between the strain gauge voltages, for a fixed voltage across the bridge, the greater the pressure difference. Output signals from the differential amplifiers 102, 104 can be applied to the microcontroller 65 integrated into the circuit board 64, and the microcontroller 65 can transmit the measured pressure data to a device external to the patient. If desired, a fully compensated Wheatstone bridge circuit can also be used to increase the sensitivity and accuracy of the pressure sensor 62. In a fully compensated bridge circuit, four strain gauges are attached to the surface of diaphragm rather than only two strain gauges.

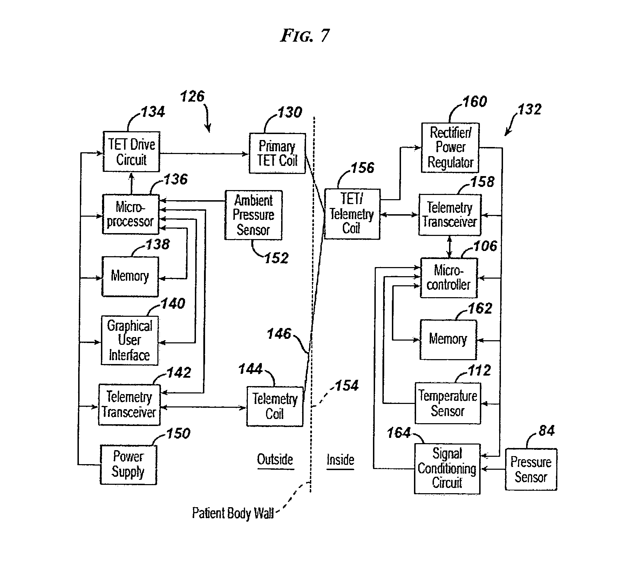

FIG. 7 illustrates one embodiment of components included in the internal and external portions 10a, 10b. As shown in FIG. 7, the external portion 10b includes a primary TET coil 130 for transmitting a power signal 132 to the internal portion 10a. A telemetry coil 144 is also included for transmitting data signals to the internal portion 10a. The primary TET coil 130 and the telemetry coil 144 combine to form an antenna, e.g., the reading device 70. The external portion 10b, e.g., disposed in the control box 90, includes a TET drive circuit 134 for controlling the application of power to the primary TET coil 130. The TET drive circuit 134 is controlled by a microprocessor 136 having an associated memory 138. A graphical user interface 140 is connected to the microprocessor 136 for inputting patient information, displaying data and physician instructions, and/or printing data and physician instructions. Through the user interface 140, a user such as the patient or a clinician can transmit an adjustment request to the physician and also enter reasons for the request. Additionally, the user interface 140 can enable the patient to read and respond to instructions from the physician and/or pressure measurement alerts.

The external portion 10b also includes a primary telemetry transceiver 142 for transmitting interrogation commands to and receiving response data, including sensed pressure data, from the implanted microcontroller 65. The primary transceiver 142 is electrically connected to the microprocessor 136 for inputting and receiving command and data signals. The primary transceiver 142 drives the telemetry coil 144 to resonate at a selected RF communication frequency. The resonating circuit can generate a downlink alternating magnetic field 146 that transmits command data to the microcontroller 65. Alternatively, the transceiver 142 can receive telemetry signals transmitted from a secondary TET/telemetry coil 114 in the internal portion 10a. The received data can be stored in the memory 138 associated with the microprocessor 136. A power supply 150 can supply energy to the control box 90 in order to power element(s) in the internal portion 10a. An ambient pressure sensor 152 is connected to microprocessor 136. The microprocessor 136 can use a signal from the ambient pressure sensor 152 to adjust the received pressure measurements for variations in atmospheric pressure due to, for example, variations in barometric conditions or altitude, in order to increase the accuracy of pressure measurements.

FIG. 7 also illustrates components of the internal portion 10a, which in this embodiment are included in the sensor housing 60 (e.g., on the circuit board 64). As shown in FIG. 7, the secondary TET/telemetry coil 114 receives the power/communication signal 132 from the external antenna. The secondary coil 114 forms a tuned tank circuit that is inductively coupled with either the primary TET coil 130 to power the implant or the primary telemetry coil 144 to receive and transmit data. A telemetry transceiver 158 controls data exchange with the secondary coil 114. Additionally, the internal portion 10a includes a rectifier/power regulator 160, the microcontroller 65, a memory 162 associated with the microcontroller 65, a temperature sensor 112, the pressure sensor 62, and a signal conditioning circuit 164. The implanted components can transmit pressure measurements (with or without adjustments due to temperature, etc.) from the sensor 62 to the control box 90 via the antenna (the primary TET coil 130 and the telemetry coil 144). Pressure measurements can be stored in the memory 138, adjusted for ambient pressure, shown on a display on the control box 90, and/or transmitted, possibly in real time, to a remote monitoring station at a location remote from the patient.

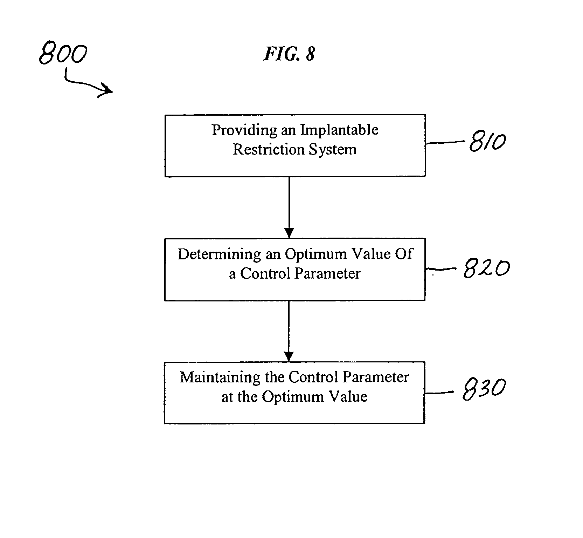

As indicated above, methods for optimizing the operation of a gastric restriction system are disclosed herein. FIGS. 8-12 illustrate a variety of methods for optimizing the operation of the restriction system 10. While the methods shown in FIGS. 8-12 are discussed with relation to the elements included in FIGS. 1A-7, a person skilled in the art will appreciate that the process can be modified to include more or fewer elements, reorganized or not, and can be performed in the restriction system 10 disclosed herein or in another, similar system having other, similar elements.

FIG. 8 illustrates one exemplary embodiment of a method for optimizing the operation of a gastric restriction system 800. The method can generally include providing an implantable restriction system 810 for forming a restriction in a patient, determining an optimum value of a control parameter 820 of the restriction system, and maintaining the control parameter at the optimum value 830 such that a result parameter of the restriction system is substantially convergent as a function of time. As indicated above, the implantable restriction system of the method 800 can have a variety of configurations. In general, the restriction system can include an adjustable restriction device that is configured to form a restriction in a patient such as, for example, the gastric restriction band 20 described above. In one exemplary embodiment, the implantable restriction system can take the form of the exemplary restriction system 10 shown and described in FIGS. 1A-7.

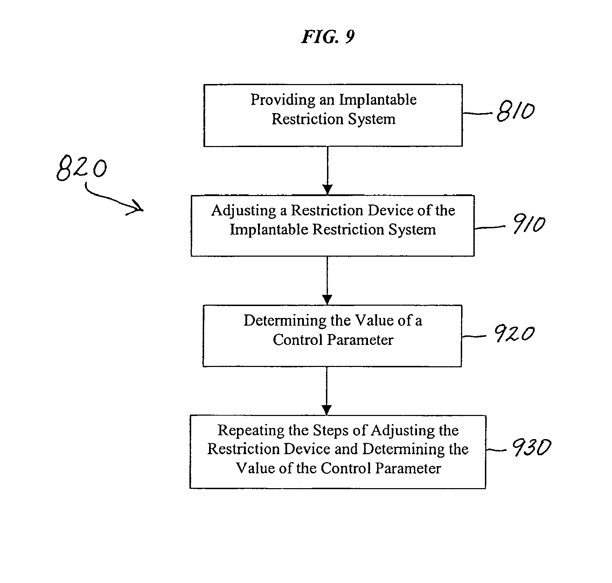

FIG. 9 illustrates one exemplary embodiment of a method for determining an optimum value of a control parameter 820. In general, a control parameter can represent an operational parameter of the implantable restriction system that can be directly controlled by a physician via adjustment of the adjustable restriction device. Examples of control parameters include, but are not limited to, a pressure within the restriction system, flow rate of a bolus into the stomach, a peristaltic pulse event or frequency, a peristaltic pulse width, a peristaltic pulse duration, and a peristaltic pulse amplitude. Determining an optimum value of a control parameter 820 can generally include adjusting the restriction device 910, determining the value of the control parameter to be optimized 920, and repeating the steps of adjusting the restriction device and determining the value of the control parameter 930 until the control parameter is substantially convergent as a function of time.

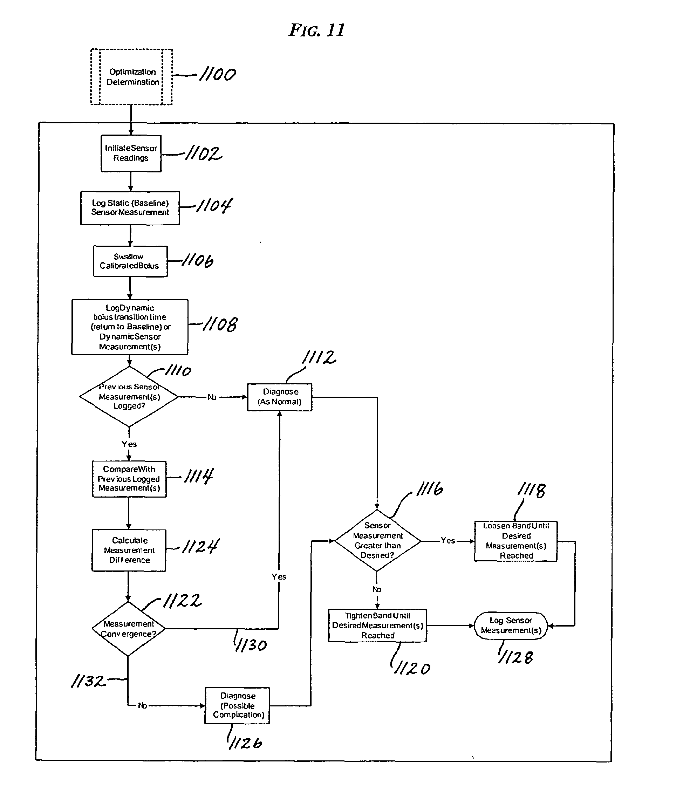

FIG. 11 illustrates the control parameter optimization process 1100 in greater detail.
Once the physician has determined which control parameter is to be optimized, the optimization procedure can be initiated by taking an initial or baseline measurement of the control parameter 1102. One skilled in the art will appreciate that a variety of methods can be used to measure a value of the control parameter. For example, in one exemplary embodiment, a value of the control parameter can be detected using a sensor disposed in the restriction system 10 such as, for example, the pressure sensor 62 described above. In particular, the pressure sensor 62 can be configured to count non-zero peristaltic pulses. After the baseline measurement is recorded 1104 by, for example, the microcontroller 65 discussed above, the patient can swallow a calibrated bolus 1106 to stimulate a peristaltic response. A dynamic sensor measurement can now be taken and recorded 1108 by the microcontroller 65. A dynamic sensor measurement can generally include, for example, measuring the value of the control parameter as the bolus moves through the esophago-gastric junction.

As shown in FIG. 11, the determined value of the control parameter (i.e., the dynamic sensor measurement of the control parameter) can be compared to a previously determined value of the control parameter 1110 and the difference between the two values can be calculated 1124. If more than one dynamic sensor measurements are recorded for the patient, the most recently recorded measurement can be compared to the last recorded dynamic measurement for the control parameter 1114. If there is only one dynamic sensor measurement recorded for the patient, the recorded measurement can be compared to a pre-determined value for the control parameter 1112. In general, the pre-determined value can be a set number or range selected and known by the physician to correspond to the successful operation of a restriction system in other patients. For example, in one exemplary embodiment, an experimentally pre-determined number of peristaltic pulses, such as 5-10 pulse counts, can serve as an initial baseline for acceptable number of peristaltic pulses indicating adequate system operation.

Regardless of whether the recorded dynamic measurement is compared to a previously recorded measurement 1114 or a pre-determined value for the control parameter 1112, the next step in the optimization procedure is the same. If the recorded dynamic sensor measurement and the previously determined value of the control parameter are substantially equal 1122, the system is operating at an optimum value 1130 and no adjustment of the restriction device is necessary. However, if the recorded dynamic sensor measurement and the previously determined value of the control parameter are not substantially equal, this can indicate a possible complication with the operation of the system. Thus, if the measured value of the control parameter and the previously determined value of the control parameter are not substantially equal 1132, the physician can diagnose the possible complication 1126 and adjust the restriction device to correct the complication. A number of factors can affect the adjustment of the band. For example, the operating parameter chosen by the physician to be the control parameter, the measured value of the control parameter, and how the control parameter is measured can all influence the adjustment of the band.

In one exemplary embodiment, the control parameter can be the peristaltic pulse duration and can be dynamically measured in seconds. If the recorded measurement of the peristaltic pulse duration is less than the previously determined value of the peristaltic pulse duration, the restriction device can be tightened 1120. Tightening the band can improve the performance of the system because a measured parameter that is less than the previously determined value generally corresponds to food passing too easily through the restriction at the esophageal-gastric junction. The restriction device can generally be tightened by increasing the pressure within the restriction system. In one embodiment, increasing the pressure within the restriction system can include increasing the fluid pressure within the closed circuit of the system. In another embodiment, increasing the pressure within the restriction system can include tightening the restriction device itself (i.e., decreasing the diameter of the restriction formed by the gastric band as it is applied to the esophageal-gastric junction). As indicated above, several factors can affect the adjustment of the band. Thus, a measured value of a control parameter that is less than a previously determined value of the control parameter does not always result in a tightening of the restriction device. Exemplary embodiments of control parameters and measurement techniques that yield a tightening of the restriction device when the measured value of the control parameter is less than the previously determined value of the control parameter include, but are not limited to, dynamic or static measurements of the pressure within the restriction system in PSI or mmHg, dynamic measurements of the peristaltic pulse event by pulse count or pulse frequency, and dynamic measurements of the peristaltic pulse duration in seconds.

Alternatively, in another exemplary embodiment, if the recorded measurement of the control parameter is greater than the previously determined value of the control parameter 1116, the restriction device can be loosened 1118. For example, in this embodiment, the control parameter can be the pressure within the restriction system and can be statically or dynamically measured in either PSI or mmHg. Loosening the band can improve the performance of the system because a measured parameter that is greater than the previously determined value generally corresponds to food either not passing or having difficulty passing through the restriction at the esophageal-gastric junction. The restriction device can be loosened by decreasing the pressure within the restriction system. Decreasing the pressure within the restriction system can include, for example, decreasing the fluid pressure within the closed circuit of the system and loosening the restriction device itself (i.e., increasing the diameter of the restriction formed by the gastric band as it is applied to the esophageal-gastric junction). As with the above embodiment, several factors can affect the adjustment of the band. Thus, a measured value of the control parameter that is greater than a previously determined value of the control parameter does not always result in a loosening of the restriction device.

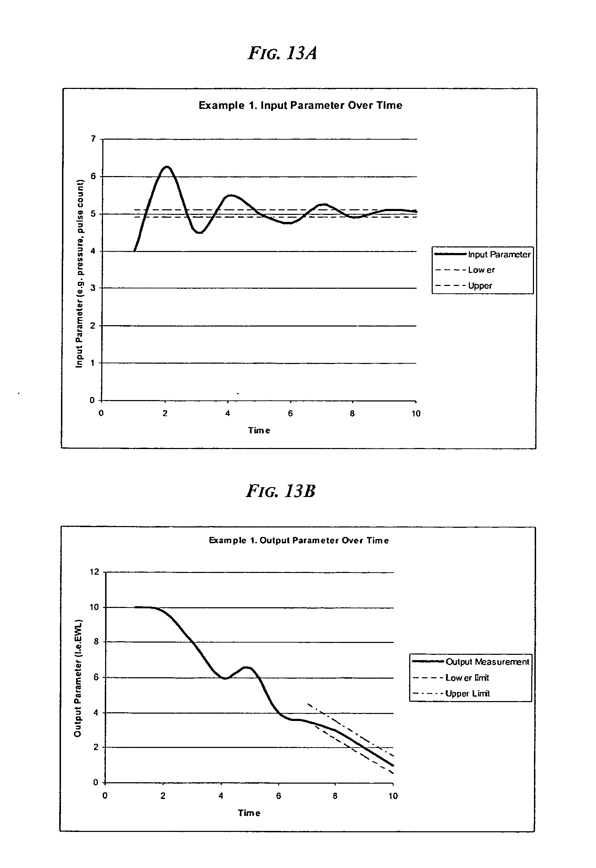

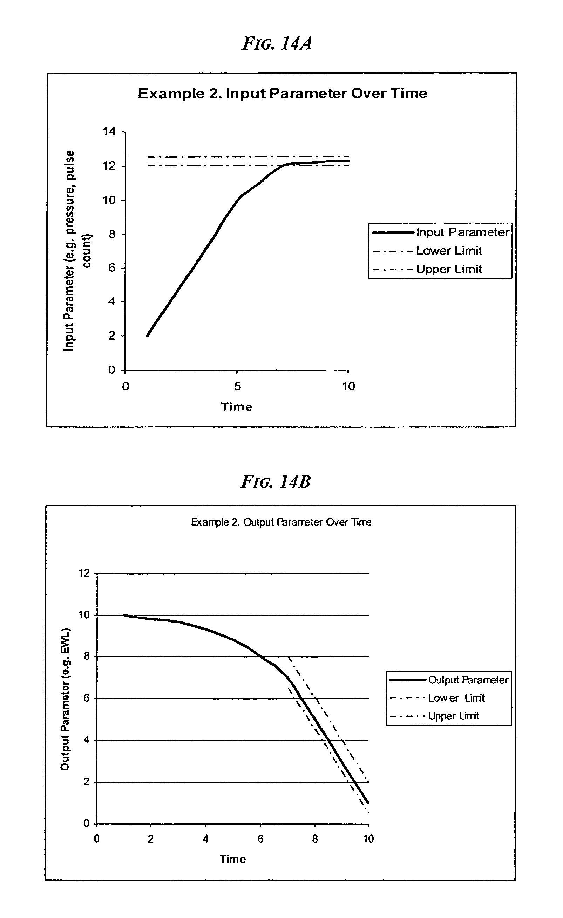

As indicated above, the steps of adjusting the restriction device and determining the value of the control parameter can be repeated 930 until the control parameter is substantially convergent as a function of time (i.e., until the control parameter substantially converges on a value over time). For example, as shown in FIG. 11, the restriction device can be adjusted 1118, 1120 until the determined value of the control parameter (i.e., the dynamic sensor measurement of the control parameter) and the previously determined value of the control parameter are substantially equal. The sensor measurements can be logged 1128 to create an optimization history for the patient. FIGS. 13A and 14A illustrate that maintaining the control parameter at a value that is substantially equal to a previously determined value of the control parameter over time can correspond to a control parameter that is substantially convergent as a function time. It is this value of the control parameter (i.e., the value of the control parameter that has substantially converged on a value over time) that can correspond to the optimum value of the control parameter for a specific patient. The terms substantially equal and substantially convergent or converges can include variations in the value of the control parameter in the range of about 5-10%.

Referring back to FIG. 8, once an optimum value of a control parameter of the restriction system is determined 820, maintaining the control parameter at the optimum value 830 can be effective to substantially converge a result parameter of the restriction system as a function of time. In general, a result parameter can represent an output result of the implantable restriction system that can be indirectly controlled by a physician via adjustment of the adjustable restriction device. Examples of result parameters include, but are not limited to, the body mass index of the patient, the weight of the patient, the change in weight of the patient, and percent excess weight lost by the patient. As shown in FIGS. 13A-14B, substantially maintaining the control parameter at an optimum value over time corresponds to a substantially convergent result parameter as a function of time. As with the control parameter, substantially convergent can include variations in the value of the result parameter in the range of about 5-10%. Substantially converging the result parameter as a function of time can be effective to optimize the operation of the restriction system, as a substantially convergent result parameter generally corresponds with steady, consistent weight loss by the patient over time.

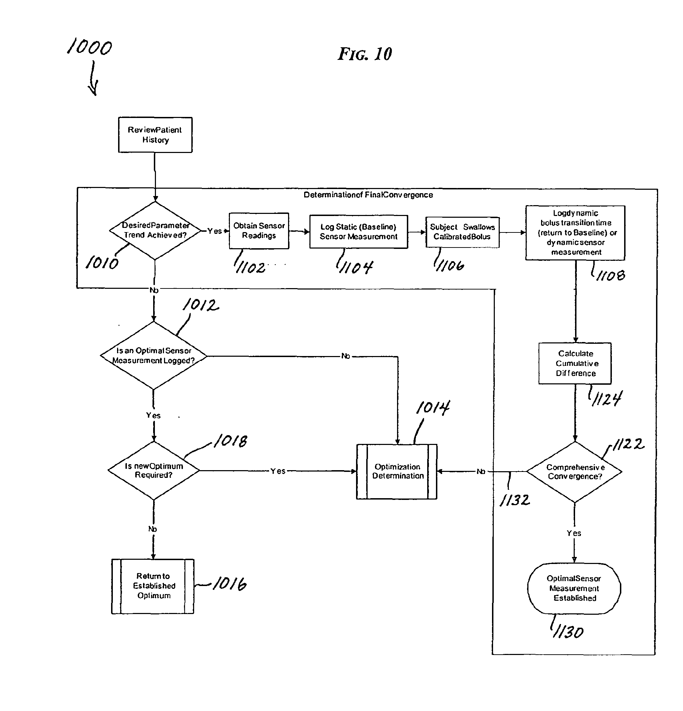

FIG. 10 cumulatively illustrates one exemplary embodiment of a method for optimizing the operation of a restriction system 1000. As shown, the first step is to determine if the desired result parameter trend is achieved 1010 (i.e., is the patient losing weight at a steady, consistent rate?). If yes, the value of a control parameter can be substantially maintained by repeating the steps of measuring the control parameter and comparing the measured value to a pre-determined value for the control parameter as described above and shown in FIGS. 9 and 11. If the desired results parameter trend is not achieved (i.e., the patient is not losing weight at a steady, consistent rate), a determination can be made as to the optimum value of a control parameter of the system 1012. If an optimum value for a control parameter has not been determined, an optimum value for the control parameter can be determined 1014 as described above and shown in FIGS. 9 and 11. If an optimum value for a control parameter has already been determined and recorded, the control parameter can be returned to its established optimum value 1016.

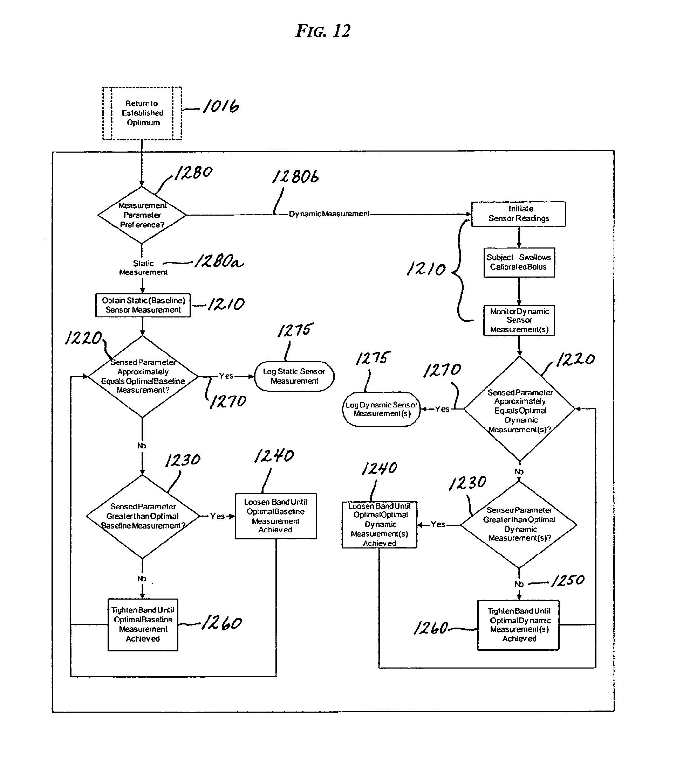

Returning the control parameter to a previously determined optimum value 1016 generally includes the steps of determining the current value of the control parameter 1210, comparing the current value of the control parameter to the previously determined optimum value for the control parameter 1220, and adjusting the restriction device accordingly. As shown in FIG. 12, the physician can first select a measurement preference 1280. For example, the physician can chose to measure the current value of the control parameter statically 1280a or dynamically 1280b. As indicated above, a dynamic measurement of the control parameter can include, for example, measuring the value of the control parameter as a bolus moves through the esophago-gastric junction. A static measurement of the control parameter can generally include measuring the "resting" value of the control parameter. For example, a static measurement of the control parameter can be taken in between mealtimes. Regardless of whether the current value of the control parameter is determined via static 1280a or dynamic 1280b measurement, the next step is to compare the measured value of the control parameter to the previously determined optimum value for the control parameter 1220. FIG. 12 illustrates three possible outcomes of the comparison 1220. In particular, if the measured value 1210 of the control parameter is substantially equal to the previously determined optimum value 1270, the control parameter is currently at its optimum value and the measured value 1210 can simply be logged 1275 as no adjustment of the restriction device is necessary. If the measured value 1210 of the control parameter is greater than the previously determined optimum value 1230, the restriction device can be loosened 1240 as described above with reference to FIG. 11. Alternatively, if the measured value 1210 of the control parameter is less than the optimum value 1250, the restriction device can be tightened 1260 as described above with reference to FIG. 11. The steps of determining the current value of the control parameter 1210, comparing the current value of the control parameter to the previously determined optimum value of the control parameter 1220, and adjusting the restriction device 1240, 1260 can be repeated until the measured value of the control parameter 1210 is substantially equal 1270 to the previously determined optimum value for the control parameter as such a comparison indicates that the control parameter has been returned to its established optimum value 1016. As noted above, the term substantially equal can include variations in the value of the control parameter in the range of about 5-10%.

Referring back to FIG. 10, in some embodiments, it may be necessary to determine a new optimum value for the control parameter 1018. For example, if a previously determined optimum value no longer corresponds with a desired result parameter trend, a new optimum value for the control parameter may need to be determined using the steps described above and shown in FIGS. 9 and 11.

In general, the methods disclosed herein for optimizing the operation of a restriction system can minimize the guesswork required by a physician for a successful restriction operation. Once an optimum value for a control parameter is determined, the physician or other person performing the system adjustments can input the same value each time without undue experimentation. Maintaining the optimum value for the control parameter can result in a convergent result parameter thereby yielding a restriction system that produces predictable weight loss. This transforms system adjustment into a repeatable process that can be performed by less skilled personnel or by an automatically adjustable restriction device.

One skilled in the art will appreciate further features and advantages of the invention based on the above-described embodiments. Accordingly, the invention is not to be limited by what has been particularly shown and described, except as indicated by the appended claims.

## Claims

1. A method for determining an optimum control parameter of a restriction system for forming a restriction in a patient, comprising:
providing an implantable restriction system for forming a restriction in a patient, the system including an adjustable restriction device configured to form a restriction in a patient;
adjusting the restriction device;
determining the value of a control parameter of the restriction system; and
repeating the steps of adjusting the restriction device and determining the value of the control parameter until the control parameter is substantially convergent as a function of time.

2. The method of claim 1, further comprising comparing the determined value of the control parameter to a previously determined value of the control parameter.

3. The method of claim 2, wherein the restriction device is adjusted if the determined value of the control parameter is not substantially equal to the previously determined value of the control parameter.

4. The method of claim 3, wherein substantially equal includes variations in the value of the control parameter in the range of about 5-10%.

5. The method of claim 2, wherein adjusting the restriction device includes tightening the restriction device if the determined value of the control parameter is less than the previously determined value of the control parameter.

6. The method of claim 2, wherein adjusting the restriction device includes loosening the restriction device if the determined value of the control parameter is greater than the previously determined value of the control parameter.

7. A method for optimizing the operation of a restriction system for forming a restriction in a patient, comprising:
providing an implantable restriction system for forming a restriction in a patient, the system including an adjustable restriction device configured to form a restriction in a patient;
determining an optimum value of a control parameter of the restriction system by adjusting the restriction device until the control parameter is substantially convergent as a function of time; and
maintaining the control parameter at the optimum value such that a result parameter of the restriction system is substantially convergent as a function of time.

8. The method of claim 1 or 7, wherein the control parameter is the pressure within the restriction system.

9. The method of claim 1 or 7, wherein the control parameter is at least one of the peristaltic pulse event, the peristaltic pulse width, the peristaltic pulse duration, the peristaltic pulse amplitude, and the flow rate of a bolus into the stomach.

10. A method for optimizing the operation of a restriction system for forming a restriction in a patient, comprising:
providing an implantable restriction system for forming a restriction in a patient, the system including an adjustable restriction device configured to form a restriction in a patient;
determining an optimum value of a control parameter of the restriction system by adjusting the restriction device until the control parameter substantially converges on a value over time; and
maintaining the control parameter at the optimum value such that a result parameter of the restriction system has a substantial convergence over time.

11. The method of claim 7 or 10, wherein determining the optimum value of the control parameter includes detecting a value of the control parameter and comparing the detected value to a previously determined value of the control parameter.

12. The method of claim 11, wherein adjusting the restriction device includes tightening the restriction device if the detected value of the control parameter is less than the previously determined value of the control parameter.

13. The method of claim 11, wherein adjusting the restriction device includes loosening the restriction device if the detected value of the control parameter is greater than the previously determined value of the control parameter.

14. The method of claim 1, 7 or 10, wherein substantially convergent includes variations in the value of the control parameter in the range of about 5-10% over time.

15. The method of claim 7 or 10, wherein substantial convergence includes variations in the value of the result parameter in the range of about 5-10% over time.
